# EUROPÄISCHE PATENTSCHRIFT

(11) **EP 1 661 540 B1**
(45) Veröffentlichungstag und Bekanntmachung des Hinweises auf die Patenterteilung: **08.07.2009**
(21) Anmeldenummer: 04028211.3
(22) Anmeldetag: 27.11.2004
(51) Int. Cl.: A61F 13/64, A61F 13/56, A61F 13/62

(54) **Wegwerfwindel**
Disposable Diaper
Couche-culotte jetable

(43) Veröffentlichungstag der Anmeldung: 31.05.2006
(73) Patentinhaber: Paul Hartmann Aktiengesellschaft, 89522 Heidenheim (DE)
(72) Erfinder: Röhrl, Wolfgang, 89542 Herbrechtingen (DE); Bandorf, Matthias, 97422 Schweinfurt (DE); Kesselmeier, Rüdiger Dr., 89542 Herbrechtingen (DE)
(74) Vertreter: Friz, Oliver

(56) Entgegenhaltungen:
- WO-A-01/43672
- WO-A-02/24134
- WO-A-20/04069122
- US-A1- 2001 034 512
- US-A1- 2002 045 881
- US-A1- 2004 236 304
- US-B1- 6 443 930

## Beschreibung

Die Erfindung betrifft eine Wegwerfwindel, insbesondere zur Inkontinentenversorgung, mit einem eine in Umfangsrichtung geschlossene Hüftöffnung der Windel bildenden Hüftgürtel, der an wenigstens einer Stelle mittels erster und zweiter Verschlussmittel öffenbar und auf sich selbst schließbar ist, und mit einem einen Vorderbereich, einen Rückenbereich und einen dazwischen liegenden Schrittbereich aufweisenden Windelhauptteil, der einen Absorptionskörper für Flüssigkeiten aufweist, wobei der Windelhauptteil mit dem Längsende seines Vorderbereichs oder seines Rückenbereichs über dritte Verschlussmittel lösbar am Hüftgürtel festlegbar ist, derart, dass ein Benutzer bei angelegtem Hüftgürtel den Windelhauptteil zwischen den Beinen hervorholen und das noch freie Längsende des Windelhauptteils am Hüftgürtel lösbar festlegen kann.

Eine derartige Wegwerfwindel ist beispielsweise aus WO-01/00129-A1 bekannt.

Derartige Wegwerfwindeln, auch häufig als Gürtelwindeln bezeichnet, bieten den Vorteil, dass ein Benutzer beim Anlegen der Windel zunächst den Hüftgürtel um die Hüften herumlegt, und üblicherweise im Bauchbereich schließen kann. Dies erfolgt, in dem der eine Hüftgürtelabschnitt mittels erster Verschlussmittel auf der körperabgewandten Seite des anderen Hüftgürtelabschnittes an dessen zweite Verschlussmittel in überlappender Konfiguration festgelegt wird. Währenddessen hängt der üblicherweise mit seinem Rückenbereich am Gürtel befestigte Windelhauptteil der Wegwerfwindel lose nach unten. Der Benutzer ergreift dann nach dem Schließen des Hüftgürtels das freihängende Ende des Windelhauptteils und führt den Windelhauptteil von hinten zwischen den Beinen hervor, um den Windelhauptteil mit seinem freien Längsende am Hüftgürtel außen lösbar festzulegen. Hierfür sind am freien Längsende die dritten Verschlussmittel, die üblicherweise als mechanische Verschlussmittel, insbesondere als Kletthaken ausgebildet sind und an der körperabgewandten Seite des Hüftgürtels vierte Verschlussmittel, die üblicherweise als schlaufenbildendes Fasermaterial, insbesondere als Vliesstoff ausgebildet sind, vorgesehen. Es versteht sich, dass das Anlegen der Wegwerfwindel auch so ausgeführt werden kann, dass nach dem Anlegen und Schließen des Hüftgürtels der frei nach unten hängende Windelhauptteil von vorn nach hinten zwischen den Beinen eines Benutzers hindurchgeführt und solchenfalls mit seinem Rückenbereich am Hüftgürtel lösbar befestigt werden kann. Es sind aber auch Wegwerfwindeln bekannt geworden, bei denen der Windelhauptteil ganz vom Hüftgürtel lösbar ist, so dass insbesondere bei stark pflegebedürftigen und immobilen Benutzern eine weitgehende Flexibilität bei der Handhabung der Wegwerfwindel gewährleistet ist.

Nachteil der bekannten Gürtelwindeln ist die mangelnde Flexibilität beim Festlegen des Windelhauptteils mit seinem freien Längsende am Hüftgürtel mittels dritter und vierter Verschlussmittel. Die dritten, mechanischen Verschlussmittel sind im Stand der Technik bislang derart ausgebildet, dass sie ihrer Primärfunktion entsprechend mit den vierten Verschlussmitteln insbesondere mit der gesamten Außenseite des den Hüftgürtel bildenden Materials haftend zusammenwirken können. Nach dem Festlegen der Hüftgürtelabschnitte zur Bildung einer geschlossenen Hüftöffnung mittels erster und zweiter Verschlussmittel verdecken die ersten Verschlussmittel jedoch einen Teil der vierten Verschlussmittel, da die ersten Verschlussmittel üblicherweise über den Seitenrand des Hüftgürtelabschnittes überstehen. Dieser Teil der vierten Verschlussmittel steht mithin als Landefläche für die dritten, mechanischen Verschlussmittel nicht mehr zur Verfügung. Die Möglichkeiten des passgenauen Festlegens des Windelhauptteils mit seinem freien Längsende am Hüftgürtel mittels dritter und vierter Verschlussmittel sind somit eingeschränkt. Dieser Nachteil ist insbesondere dann bedauerlich, wenn die ersten und dritten Verschlussmittel mit der gesamten Außenseite des den Hüftgürtel bildenden Materials haftend zusammenwirken können, wobei in diesem Fall zweite und vierte Verschlussmittel zusammenfallen.

WO-A-01/43672 und WO-A-2004/069122 zeigen nicht gattungsgemäße Windeln, bei denen sich das anmeldungsgemäße Problem nicht stellt, da die ersten Verschlussmittel innerhalb der Erstreckung der Hüftgürtelabschnitte vorgesehen sind.

Aufgabe vorliegender Erfindung ist somit, diesen Nachteil mangelnder Flexibilität des passgenauen Anlegens einer Gürtelwindel mit dritten, mechanischen Verschlussmitteln zu überwinden.

Diese Aufgabe wird bei einer Wegwerfwindel der genannten Art erfindungsgemäß dadurch gelöst, dass die dritten, mechanischen Verschlussmittel auch an der körperabgewandten Seite der ersten Verschlussmittel lösbar festlegbar sind.

Es wird also erfindungsgemäß sichergestellt, dass die dritten, mechanischen Verschlusselemente nicht nur mit den vierten Verschlusselementen des Hüftgürtels eine Haftverbindung eingehen können, sondern dass auch die körperabgewandte Seite der ersten Verschlussmittel eine haftende Verbindung mit den dritten mechanischen Verschlusselementen eingehen können. Diese Haftverbindung soll so groß sein, dass die Primärfunktion der Verschlussmittel, also der sichere Halt der Windel in Gebrauch gewährleistet ist.

Diese Haftverbindung kann in an sich beliebiger Weise realisiert werden, zum Beispiel durch Haftklebstoffe. Vorteilhaft ist hingegen insbesondere, wenn die körperabgewandte Seite der ersten Verschlussmittel zumindest abschnittsweise aus einem Fasermaterial wie insbesondere einem Vliesmaterial, vorzugsweise einem Kardenvlies oder einem Spinnvlies, oder einem textilen Material gebildet ist.

In Weiterbildung der Erfindung wird vorgeschlagen, die ersten und/oder die dritten Verschlussmittel zumindest abschnittsweise elastisch auszubilden. Dies erhöht nochmals die Möglichkeiten, die Wegwerfwindel passgenau anzupassen. Der elastische Abschnitt der jeweiligen Verschlussmittel wird vorteilhaft aus einer elastischen Folie oder einem ein- oder mehrschichtigen elastischen Vlies oder einem elastischen Vliesfolienlaminat gebildet. Hierbei kann beispielsweise eine elastische Folienkomponente in gedehntem Zustand auf einer an sich unelastischen Vlieskomponente fixiert sein, so dass das gebildete Vliesfolienlaminat elastische Eigenschaften aufweist. Die Vlieskomponente sollte im Falle der ersten Verschlussmittel dann vorzugsweise körperabgewandt zu liegen kommen und solchenfalls insbesondere derart ausgebildet ist, dass die dritten Verschlussmittel daran festlegbar sind.

Um eine ausreichend große Fläche für die Haftverbindung der ersten und zweiten Verschlussmittel zu gewährleisten, stehen die ersten Verschlussmittel über den Seitenrand des Hüftgürtels um mindestens 10 mm, insbesondere um mindestens 20 mm, weiter insbesondere um mindestens 30 mm und weiter insbesondere um mindestens 40 mm über. Die Erstreckung der ersten Verschlussmittel in Längsrichtung der Gürtelwindel beträgt insbesondere mindestens 15 mm, weiter insbesondere mindestens 20 mm, weiter insbesondere mindestens 25 mm, weiter insbesondere mindestens 30 mm und weiter insbesondere mindestens 35 mm. Mit der vorliegenden Erfindung ist es möglich, die Größe der ersten Verschlussmittel ausschließlich nach der optimalen Erfüllung ihrer Primärfunktion, nämlich der ausreichenden Haftkraft beim Zusammenwirken mit den zweiten Verschlussmitteln zu wählen. Auch ein sehr großes erstes Verschlussmittel schränkt den Anwender hinsichtlich der Flexibilität des nachfolgenden Festlegens des Windelhauptteils mit seinem freien Längsende am Hüftgürtel mittels dritter Verschlussmittel nicht ein, da die dritten Verschlussmittel auch auf der körperabgewandten Seite der ersten Verschlussmittel ausreichend stark haften können, also den sicheren Halt der Windel in Gebrauch gewährleisten können.

In weiterer Ausbildung des Erfindungsgedankens werden die Materialien der Verschlussmittel derart aufeinander abgestimmt, dass die Haftkraft als Scherkraft wie nachfolgend beschrieben gemessen zwischen der körperabgewandten Seite des ersten Verschlussmittels und dem drittem Verschlussmittel geringer ist als die Haftkraft als Scherkraft zwischen der körperzugewandten Seite des ersten Verschlussmittels und dem zweiten Verschlussmittel. Hierdurch ist sichergestellt, dass sich während des Gebrauchs der Windel bei entsprechender Krafteinwirkung im schlimmsten Fall zunächst nur das festgelegte Ende des Windelhauptteils vom Hüftgürtel löst, der Hüftgürtel selbst aber verschlossen bleibt, so dass sich die Gürtelwindel nicht vollständig vom Körper löst.

In besonders vorteilhafter Ausbildung der vorliegenden Erfindung beträgt die Haftkraft zwischen der körperzugewandten Seite des ersten Verschlussmittels und dem zweiten Verschlussmittel mindestens 5 N/25mm, insbesondere mindestens 10N/25mm, weiter insbesondere mindestens 15N/25mm, weiter insbesondere mindestens 20N/25mm, weiter insbesondere mindestens 25N/25mm, weiter insbesondere mindestens 30N/25mm, weiter insbesondere mindestens 35N/25mm, weiter insbesondere mindestens 40N/25mm, weiter insbesondere mindestens 45N/25mm und weiter insbesondere mindestens 50N/25mm. Vorteilhafterweise beträgt die Haftkraft zwischen der körperzugewandten Seite des ersten Verschlussmittels und dem zweiten Verschlussmittel aber weiniger als 80N/25mm, insbesondere weniger als 70N/25mm ist.

Die Haftkraft zwischen der körperabgewandten Seite des ersten Verschlussmittels und dem drittem Verschlussmittel beträgt vorteilhafterweise mindestens 2 N/25mm, insbesondere mindestens 3,5 N/25mm, weiter insbesondere mindestens 5 N/25mm, weiter insbesondere mindestens 10N/25mm, weiter insbesondere mindestens 15N/25mm, weiter insbesondere mindestens 20N/25mm, weiter insbesondere mindestens 25N/25mm, weiter insbesondere mindestens 30N/25mm, weiter insbesondere mindestens 35N/25mm, weiter insbesondere mindestens 40N/25mm, weiter insbesondere mindestens 45N/25mm und weiter insbesondere mindestens 50N/25mm.

Vorteilhafterweise beträgt die Haftkraft zwischen der körperabgewandten Seite des ersten Verschlussmittels und dem drittem Verschlussmittel aber weniger als 70N/25mm, insbesondere weniger als 60N/25mm ist.

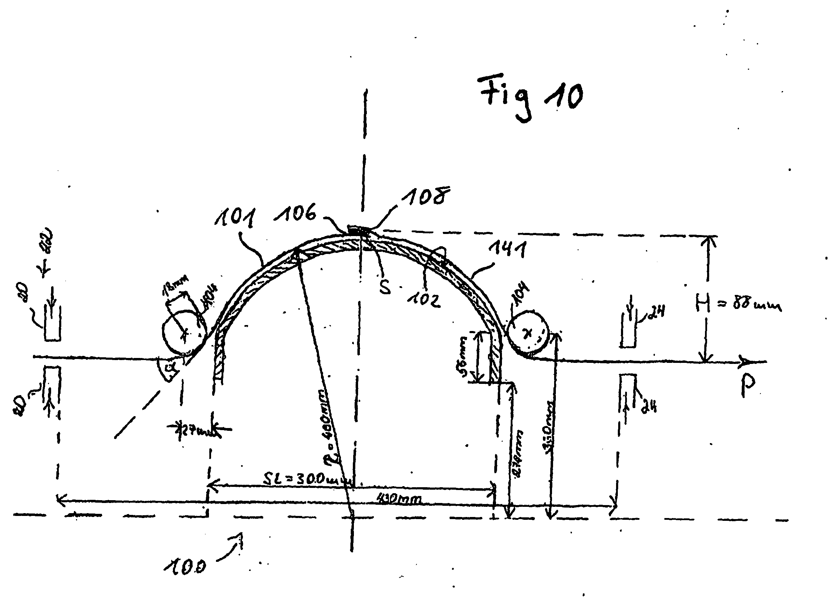

Nachfolgend wird eine Prüfmethode für die Ermittlung der Haftkräfte bei Scherbeanspruchung angegeben. Für die Durchführung der Prüfmethode kann ein Zugprüfgerät Typ Z010 / TN 2S, Messdose 100 N, erhältlich bei der Firma Zwick GmbH & Co KG, Ulm, Deutschland, mit einer Klemmbackenbreite zum Einspannen des Prüflings von 60 mm verwendet werden. Bei der Durchführung der Prüfmethode wird, wie Figuren 10-11 dies veranschaulichen, das zu prüfende Verschlusssystem mit einer ersten, beispielsweise schlaufenbildenden Komponente 106 und einer darauf haftenden zweiten beispielsweise hakenbildenden Komponente 108 über eine gekrümmte Fläche gelegt, welche eine Rundung des Bauchbereichs eines Benutzers simulieren soll (siehe Figur 10). Zur Verbindung der Verschlussmittel mit den Klemmbacken des Zugprüfgeräts wird ein biegsames Substrat 141,101, beispielsweise ein einseitiges Klebeband einer bevorzugten Breite von 25 mm, das unter der Bezeichnung STA 306 bei der Fa, 3M Deutschland GmbH ansässig in Neuss erhältlich ist, verwendet. Das Klebeband ist aus Polypropylen, seine Oberfläche ist beschichtet durch ein urethanmodifiziertes Siliconpolymer. Das Flächengewicht des Haftklebstoffauftrags beträgt 23 g/m². Der über die gekrümmte Fläche gelegte Prüfling, bestehend aus aufeinander haftenden flächenhaften Abschnitten der Verschlussmittel 106, 108, wird durch Verwendung des Zugprüfgeräts auf Zug beansprucht, woraus sich eine scherende Beanspruchung der aneinander haftenden flächenhaften Abschnitte ergibt.

### Probenvorbereitung:

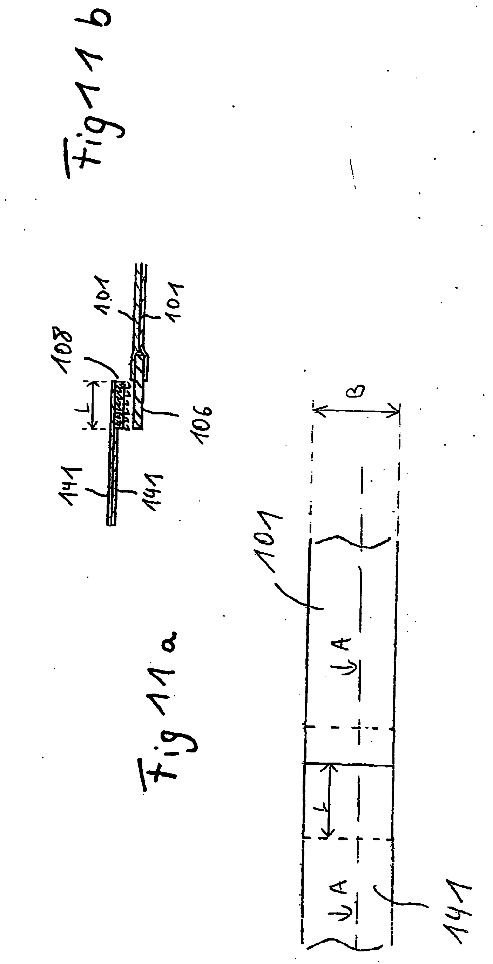

Die zu verwendenden Verschlussmittel werden über 24 h bei 23° C und 50 % relativer Luftfeuchte konditioniert. Es werden Prüflinge 106, 108 einer Größe von 25 x 30 mm (Breite B x Länge L) aus den Verschlussmittelmaterialien ausgestanzt und sandwichartig zwischen die Enden zweier einseitiger Klebebänder 101, 141 einer Breite von 25 mm, die gegeneinander geklebt sind, angeordnet bzw. fixiert, so dass sich ein Überstand der zu prüfenden Verschlussseite des jeweiligen Verschlussmittelmaterials von 25 x 20 mm (Breite B x Länge L) ergibt (siehe Figuren 11a, 11b). Sollten wie im dargestellten Fall der Figuren 11a, 11b hakenbildende Verschlussmaterialien zu prüfen sein wird vorgeschlagen, die hakenbildende Komponente des Verschlussmittels 108 mit den Abmessungen von 25 x 20 mm auszustanzen und durch zwei mit ihren Klebeflächen gegeneinander geklebte einseitige Klebebänder 101 derart zu fixieren, dass das obere Klebeband die Rückseite des flächenhaften Abschnittes überfängt und das untere Klebeband bündig an den flächenhaften Abschnitt angrenzt (siehe Figur 11b).

Die so vorbereiteten Abschnitte der Verschlussmittelmaterialien werden nachfolgend vollflächig übereinander gelegt (Figuren 11a, 11b). Sollten die Verschlusselemente der Verschlussmittelmaterialien eine bevorzugte Richtung aufweisen, so erfolgt hierbei die Ausrichtung so wie es in Gebrauch der Windel zu erwarten wäre.

Die aufeinander gelegten flächenhaften Abschnitte werden durch viermaliges Anrollen mit einer 50 mm breiten und im Durchmesser 100 mm starken Rolle mit glatter Oberfläche und einem Rollengewicht von 5 kg miteinander verbunden, wobei die Anrollgeschwindigkeit 20-100 mm/sec beträgt.

### Prüfverfahren:

Das eine Ende der wie vorstehend beschrieben verlängerten Verschlussmittelmaterialien wird in die untere Klemmbacke des Zugprüfgeräts mittig zentriert eingespannt, und das gegenüberliegende Ende der der wie vorstehend beschrieben verlängerten Verschlussmittelmaterialien wird in die bewegliche obere Klemmbacke des Zugprüfgeräts ebenfalls zentriert eingespannt.

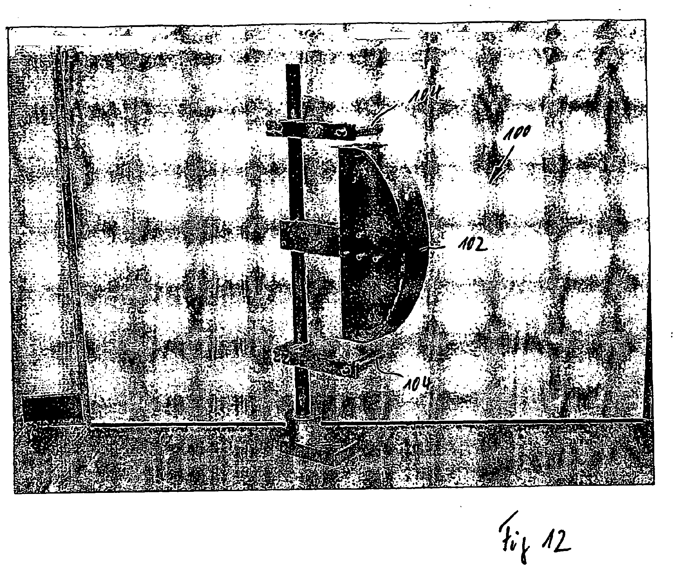

Der so eingespannte Prüfling wird über die aus Figuren 10, 12 ersichtliche Vorrichtung 100, welche den Bauch- oder Hüftbereich eines Benutzers simulieren soll, gelegt. Diese Vorrichtung 100 ist in Figur 12 perspektivisch dargestellt. Man erkennt eine bogenförmig gekrümmte Fläche 102 aus poliertem Stahl mit einer Rautiefe von 5 bis 25 µm und mit einem Krümmungsradius R von zumindest abschnittsweise 400 mm und einer Sehnenlänge SL von 300 mm. Des Weiteren sind oberhalb und unterhalb der gekrümmten Fläche 102 Umlenkrollen 104 mit einem Durchmesser von 18 mm vorgesehen, welche den über die gekrümmte Fläche gelegten Prüfling in die vertikale Richtung um H = 88 mm umlenken, wo er dann mit Klemmen 20, 24 des nicht dargestellten Zugprüfgeräts verbunden wird. Die Umlenkung erfolgt um einen Winkel α von 60°. Hierdurch wird der Abzugswinkel im Wesentlichen tangential zu der gekrümmten Fläche und konstant gehalten. Die aufeinander gelegten flächenhaften Abschnitte 106, 108 der Komponenten des Verschlussmittels werden in Bezug auf die gekrümmte Fläche 102 so positioniert, dass die obere Komponente mittig zentriert im Scheitelpunkt S der gekrümmten Fläche 102 zu liegen kommt.

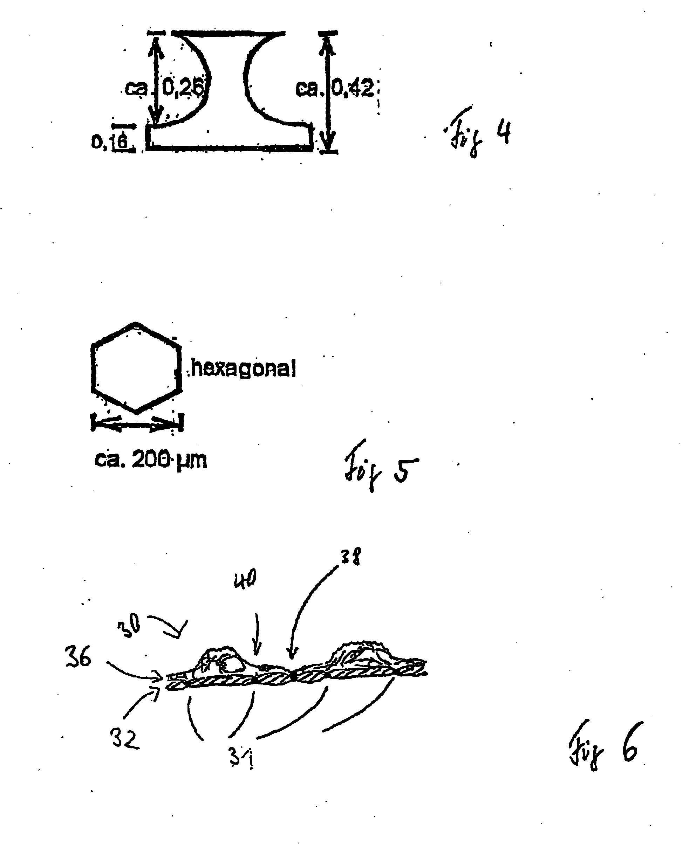

Es wird dann die bewegliche Klemme 24, die mit der oberen Komponente verbunden ist, mit der nachfolgend angegebenen Prüfgeschwindigkeit in Pfeilrichtung P bewegt, und es wird währenddessen die dabei auftretende Zugkraft zwischen den Klemmen ermittelt. Die Prüfparameter sind:
- Prüfgeschwindigkeit: 300 mm/min
- Einspannlänge des Prüflings: 430 mm (s. Figur 4)
- Messweg: Strecke bis zur Ablösung der Verschlussmittelkomponen ten voneinander
- Vorkraft: 0,2 N
- Prüfanzahl: n ≥ 6.

Die Auswertung erfolgt dergestalt, dass die bis zum voneinander Ablösen der Verschlussmittel ermittelte Maximalkraft gerundet auf zwei Dezimalstellen in N (Newton) notiert wird und in Form eines Mittelwerts der n-Messungen und Angabe der Standardabweichung sowie eines Minimalwerts und eines Maximalwerts angegeben wird.

In Weiterbildung der Erfindung wird vorgeschlagen dass der Hüftgürtel von einem einstückigen Materialabschnitt gebildet ist, der an den Windelhauptteil angefügt ist.

Es wird vorzugsweise also ein in Querrichtung oder Hüftumfangsrichtung einstückig durchgehender Materialabschnitt an den Windelhauptteil zur Bildung des Hüftgürtels angefügt. Aufgrund der flächenhaften Anfügung werden die Zugkräfte nicht in den Windelhauptteil eingeleitet sondern können ganz von dem reißfesten Gürtelmaterial aufgenommen werden. Dies eröffnet bei der Auswahl der den Hauptteil bildenden Chassismaterialien eine größere Flexibilität. Außerdem sind die Anforderungen an die Stabilität der Anfügung des Gürtels an den Hauptteil geringer. Der den Gürtel bildende Materialabschnitt kann als ein einziger Längsabschnitt einer in der Maschinenrichtung zugeführten Flachmaterialbahn erhalten werden.

In vorteilhafter Ausführung der Erfindung ist der Hüftgürtel beidseits einer Längsmittelachse um jeweils mindestens eine, insbesondere mindestens zwei, weiter insbesondere mindestens drei in einer Längsrichtung der Windel erstreckte Falzlinien auf sich selbst gefaltet.

In weiterer Ausbildung des Erfindungsgedankens erweist es sich als vorteilhaft, wenn der Hüftgürtel in der beidseits gefalteten Konfiguration lösbar fixiert ist, so dass er sich innerhalb der schnelllaufenden Herstellungsmaschine nicht unbeabsichtigt entfaltet oder aufflattert. Die Faltung und lösbare Fixierung des Hüftgürtels in der gefalteten Konfiguration ermöglicht in vorteilhafte Weise die Anfügung des sehr langen Hüftgürtels an den Hauptteil in der Herstellungsmaschine.

In Weiterbildung dieses Erfindungsgedankens erweist es sich als vorteilhaft, wenn die lösbare Fixierung durch Fügestellen oder Fügebereiche zwischen den aufeinandergefalteten Teilabschnitten des den Hüftgürtel bildenden Materialabschnitts gebildet ist. Es wäre aber auch möglich, dass die gefaltete Konfiguration durch sonstige Haltemittel, etwa einen lösbaren Tapeabschnitt, lösbar fixiert ist.

Die vorerwähnte und vorzugsweise in einem Zug zu lösende Fixierung der aufeinander gefalteten Teilabschnitte des an den Hauptteil angefügten den Hüftgürtel bildenden Materialabschnitts kann beispielsweise durch kalte Verprägung, oder durch Verprägung unter Anwendung von Temperatur (Thermoverschweißung), durch Vernadelung, insbesondere Heißvernadelung, oder durch Ultraschallverschweißung oder Laserverschweißung oder ähnliche gleichwirkende Fügemethoden erreicht werden.

Die lösbare Fixierung der aufeinander gefalteten Teilabschnitte des Materialabschnitts aneinander und möglicherweise auch an dem Hauptteil ist vorzugsweise durch mehrere im Wesentlichen punktförmige Fügestellen ausgebildet. Eine punktförmige Fügestelle der vorstehend erwähnten Art bedeutet, dass die Fügestelle eine Fläche (in Projektion auf die X-Y-Ebene des Hauptteils) von weniger als 5 mm², insbesondere von weniger als 2 mm² und weiter insbesondere von weniger als 1 mm² aufweist. Die Fügestellen müssen nicht streng punkt- oder kreisförmig sein. Denkbar und vorteilhaft sind auch von der Punkt- oder Kreisform abweichende Formen wie Dreieck-, Viereck-, Vieleck-, oder Ovalformen. Vorzugsweise ist die lösbare Fixierung der aufeinander gefalteten Teilabschnitte der Materialabschnitte aneinander durch thermisch oder durch Ultraschall erzeugte, vorzugsweise punktförmige Fügestellen ausgebildet.

Nach einer weiteren bevorzugten Ausführungsform der erfindungsgemäßen Gürtelwindel steht der einstückige Materialabschnitt in gefalteter Konfiguration mit einem Anfassbereich an seinem freien Ende über einen Längsseitenrand des Windelhauptteils in Querrichtung um insbesondere mindestens 5 mm, weiter insbesondere um mindestens 20 mm, weiter insbesondere um mindestens 30 mm, weiter insbesondere um mindestens 40 mm, weiter insbesondere um mindestens 50 mm und weiter insbesondere um mindestens 60 mm vor. Der Anfassbereich kann insbesondere vom jeweiligen freien Ende des Hüftgürtels gebildet sein.

Vor der Entfaltung des einstückigen Materialabschnitts sind die Anfassbereiche vorzugsweise in Querrichtung nach außen gewandt, also voneinander und von einer Längsmittelachse des auf einer ebenen Unterlage ausgebreiteten Windelhauptteils voneinander weggewandt, so dass sie auf bequeme Weise mit der linken Hand eines Benutzers von links und mit der rechten Hand von rechts ergreifbar sind. Die Gürtelwindel erweist sich insbesondere bei der Anwendung bei stark pflegebedürftigen Personen als besonders vorteilhaft. So wird die Gürtelwindel beispielsweise häufig an pflegebedürftige Patienten angelegt, während diese sich in Seitenlage befinden. Hierbei muss der über den Hauptteil seitlich überstehende Materialabschnitt unter dem Patienten hindurch geführt werden. Dieser Vorgang des unter dem Patienten Hindurchführens ist mit dem lösbar fixierten gefalteten Materialabschnitt, welcher den Gürtel bildet, nun wesentlich vereinfacht.

Die Erstreckung des den Hüftgürtel bildenden Materialabschnitts im entfalteten Zustand in Querrichtung über den Längsrand des Hauptteils hinaus beträgt vorzugsweise wenigstens 200 mm, insbesondere wenigstens 300 mm, weiter insbesondere wenigstens 400 mm, weiter insbesondere wenigstens 500 mm, weiter insbesondere wenigstens 600 mm und weiter insbesondere wenigstens 700 mm.

Seine Erstreckung in Längsrichtung beträgt vorzugsweise 30 bis 150 mm, insbesondere 40 bis 130 mm, weiter insbesondere 50 bis 125 mm, weiter insbesondere 60 bis 120 mm weiter insbesondere 70 bis 115 und weiter insbesondere 75 bis 110 mm.

Der den Hüftgürtel bildende Materialabschnitt ist vorzugsweise unlösbar und insbesondere an eine Außenseite des Hauptteils angefügt. Diese Anfügung kann in an sich beliebiger Weise ausgeführt sein. Der insbesondere einstückige Materialabschnitt kann indessen in vorteilhafter Weise mittels eines nur bereichsweisen Kleberauftrags an den Windelhauptteil angefügt sein. Insbesondere erweist es sich als vorteilhaft, wenn der Kleber nicht bis zu dem Rand der gegeneinander anliegenden flächenhaften Erstreckung des Materialabschnitts und des Hauptteils aufgebracht ist, so dass ein Randumfangsbereich dieses Verbunds kleberfrei bleibt. Dies hat den Vorteil, dass solchenfalls beim Laminieren kein Kleber zwischen den Lagen nach außen gedrückt wird.

Der insbesondere einstückige an den Hauptteil angefügte, den Hüftgürtel zumindest abschnittsweise bildende Materialabschnitt ist vorzugsweise aus einem Vliesstoff gebildet, insbesondere und vorzugsweise können Spunbond-Materialien (S) oder Spunbond-Meltblown-Materialien (SM), oder beidseitig mit Spunbond-Materialien versehene Meltblown-Schichten (SMS) oder auch kardierte und/oder wasservernadelte Vliesmaterialien Verwendung finden. Hierdurch ist möglich, den Hüftgürtel zum einen hautfreundlich, zum anderen großflächig als Ländefläche für die ersten und dritten Verschlussmittel auszubilden, also als einen großflächigen Bereich auszubilden, der als zweites und gleichzeitig als viertes Verschlussmittel für eine Haftverbindung mit der körperzugewandten Seite der ersten Verschlussmitteln und den dritten Verschlussmitteln zur Verfügung steht. Vliesstofflaminate, also insbesondere zweilagige, dreilagige oder mehrlagige Kombinationen der vorgenannten Vliesstoffe, können ebenfalls verwendet werden. Die Verbindung der einzelnen Lagen kann durch an sich übliche und bekannte Verfahren, beispielsweise durch thermische Fügeverfahren wie Verschweißung, insbesondere Laserverschweißung, Hotmelt, air-through, Heißkalander- oder Ultraschallverprägung erfolgen; auch die kalte Verpressung, Vernadelung, Vernähung oder Verklebung von Vliesstoffmaterialien ist denkbar. Auch die Verbindung mit textilen Geweben, Gewirken oder Gestricken, also mit eine textile Bindung im weitesten Sinne aufweisenden Materialien ist denkbar. Auch Filme aus thermoplastischen und insbesondere elastischen Materialien sind verwendbar, und zwar auch als mehrlagige Filmlaminate. Vorteilhafterweise können auch Vlies/Folienlaminate zum Einsatz kommen, die wenigstens eine Filmlage und wenigstens eine Vlieslage oder wenigstens eine Lage eines textilen Materials umfassen. Filmlagen werden insbesondere zur Realisierung von elastischen Bereichen des Hüftgürtels eingesetzt. Die Verbindung der Lagen kann wiederum durch die vorgenannten Verfahren erfolgen.

Als ein besonders bevorzugtes, den Hüftgürtel zumindest abschnittsweise bildende Material hat sich ein schlaufenbildendes Vliesstoffmaterial erwiesen, dessen in Gebrauch körperabgewandt angeordnete erste Oberseite erste größere ungebundene Bereiche umfasst, die voneinander beabstandet inselartig angeordnet sind, wobei diese ersten größeren ungebundenen Bereiche von gebundenen Konturen begrenzt sind und außerhalb der Begrenzung von zweiten kleineren ungebundenen Bereichen umgeben sind und durch diese zweiten kleineren ungebundenen Bereiche voneinander beabstandet sind.

Die größeren ungebundenen Bereiche stehen zur Ausbildung einer mechanisch wirkenden Verschlussfunktion primär zur Verfügung. Sie sind durch eine gebundene Kontur begrenzt oder definiert, die insbesondere durch linienförmige Prägung, insbesondere eine Thermoprägung, die insbesondere durch Ultraschallschweißen oder Kalanderprägung erzeugt ist, gebildet sein kann, wobei diese Prägelinien durchgehend oder unterbrochen sein können, insbesondere auch punktiert oder strichpunktiert ausgebildet sein können. Außerhalb dieser gebundenen Kontur sind aber zwischen diesen ersten ungebundenen inselartigen Bereichen weitere kleinere ungebundene Bereiche oder Gebiete vorgesehen, welche die ersten größeren ungebundenen Bereiche voneinander trennen. Wenn in diesem Zusammenhang von zweiten kleineren ungebundenen Bereichen oder Gebieten die Rede ist, so soll hierunter verstanden werden, dass die inselartigen größeren Bereiche entweder derart nah beieinander oder aneinander angeordnet sind oder die zweiten kleineren ungebundenen Bereiche ihrerseits gebundene Teilbereiche umschließen, dass es nicht möglich ist, eine größtmögliche, noch einbeschriebene Kreisfläche der ersten größeren ungebundenen Bereiche in den zweiten ungebundenen Bereichen aufzunehmen oder einzulegen.

Mit diesem Vliesstoffmaterial wird einerseits durch die ersten größeren ungebundenen inselartigen Bereiche ein insgesamt und auch jeweils für sich betrachtet ausladender und hinreichend großer Verankerungsbereich für die hakenbildende Komponente eines mechanischen Verschlussmittels zur Verfügung gestellt. Die weiteren zweiten ungebundenen Bereiche zwischen den inselartigen ersten ungebundenen Bereichen tragen aber auch zu einer möglichst kontinuierlichen Verankerungsmöglichkeit mit den hakenbildenden Komponenten eines mechanischen Verschlussmittels bei. Eine gleichwohl hinreichende Verfestigung des schlaufenbildenden Vliesstoffmaterials wird durch die die ersten ungebundenen Bereiche begrenzenden gebundenen Konturen erreicht und gegebenenfalls unterstützt durch weitere gebundene Teilbereiche, welche ihrerseits von den zweiten ungebundenen Bereichen umgeben zwischen den ersten größeren Bereichen angeordnet sind.

Im Hinblick auf eine gute Festigkeit und eine gute Einbindung der Fasern des Vliesstoffmaterials erweist es sich als vorteilhaft, wenn - wie bereits erwähnt - die zweiten kleineren ungebundenen Bereiche ihrerseits gebundene Teilbereiche umschließen. Diese Teilbereiche, die ebenfalls durch Prägung, insbesondere Thermoprägung, gebildet werden können, haben an sich beliebige Form, wobei sich eine Ausbildung der Teilbereiche in Form verhältnismäßig kurzer und schmaler Segmente als vorteilhaft erweist, deren Längserstreckung etwa das Zweibis Zehnfache, insbesondere das Zwei- bis Achtfache ihrer Breite beträgt.

Des Weiteren erweist es sich im Hinblick auf eine gute Einbindung der Fasern in das Vliesstoffmaterial als vorteilhaft, wenn jede gerade Verbindungslinie zwischen den ersten größeren ungebundenen inselartigen Bereichen stets durch eine gebundene Kontur oder einen gebundenen Teilbereich, der zwischen den ersten größeren Bereichen angeordnet ist, verläuft.

Die gebundenen Konturen weisen vorteilhafterweise Prägelinien oder Prägelinienabschnitte einer Breite von 0,2 bis 1 mm, vorzugsweise von 0,2 bis 0,8 mm und weiter vorzugsweise von 0,3 bis 0,6 mm auf. Sie haben vorteilhafterweise eine Tiefe von 0,4 bis 1,5 mm, vorzugsweise von 0,4 bis 0,9 mm, weiter vorzugsweise von 0,4 bis 0,8 mm und weiter vorzugsweise von 0,5 bis 0,7 mm.

Es hat sich des Weiteren als vorteilhaft erwiesen, wenn die ersten größeren ungebundenen inselartigen Bereiche eine Abmessung, insbesondere einen Durchmesser eines in den Bereich eingeschriebenen Kreises, von 2 bis 15 mm, vorzugsweise von 3 bis 10 mm, weiter vorzugsweise von 3 bis 8 mm und besonders bevorzugtermaßen von 4 bis 7 mm aufweisen.

Die ersten größeren ungebundenen inselartigen Bereiche haben vorzugsweise einen Flächenanteil von 5 - 75 %, insbesondere von 5 - 60 %, insbesondere von 10 - 50 %, insbesondere von 10 - 45 %, insbesondere von 15 - 45 %, insbesondere von 20 - 40 % und weiter insbesondere von 30 - 40 % der Gesamtfläche der ersten Oberseite. Es wurde erkannt, dass die ersten größeren ungebundenen inselartigen Bereiche in der Summe auch einen verhältnismäßig moderaten Anteil der gesamten Oberfläche ausmachen können, da die Verschlussfunktion zusätzlich von den zwar kleineren ungebundenen Bereichen oder Gebieten zwischen den ersten größeren ungebundenen Bereichen unterstützt wird.

Die ersten größeren ungebundenen inselartigen Bereiche haben keine notwendigerweise bestimmte Form, es erweist sich jedoch als vorteilhaft, wenn sie kreisförmig oder oval oder dreieckig oder vieleckig, vorzugsweise sechseckig oder achteckig, ausgebildet sind. Durch diese bevorzugten Strukturen lässt sich ein flächenmäßig ausladender Ankerbereich für die hakenbildende Komponente des Verschlussmittels darstellen. Es zeigt sich nämlich, dass die Verschlussfunktion durch verhältnismäßig große inselartige Bereiche, deren Abmessung vorzugsweise in allen Richtungen gleich ist, so wie dies beim Kreis oder regelmäßigen Vieleck, insbesondere Sechseck der Fall ist, besonders effektiv ist.

Im Hinblick auf eine Anordnung der ersten inselartigen Bereiche hat es sich als besonders zweckmäßig erwiesen, wenn diese wenigstens 1 mm, vorzugsweise wenigstens 1,5 mm, insbesondere wenigstens 2 mm und weiter insbesondere wenigstens 2,5 mm und besonders bevorzugtermaßen wenigstens 3 mm voneinander beabstandet sind. Sie sind jedoch vorzugsweise höchstens 10 mm, insbesondere höchstens 5 mm voneinander beabstandet.

Eine gute und ausreichende Stabilität des Vliesmaterials, d. h. eine gute Einbindung der Fasern und damit eine geringe Zerstörungsanfälligkeit des Vliesstoffmaterials lässt sich erreichen, wenn der gesamte Flächenanteil gebundener Bereiche vorzugsweise 10 bis 60 %, vorzugsweise 15 bis 40 %, weiter vorzugsweise 15 bis 30 %, insbesondere 15 bis 25 % und in besonders vorteilhafter Weise 19 bis 22 % der Gesamtfläche der ersten Oberfläche beträgt. Wenn hier von gebundenen Bereichen die Rede ist, so werden hierunter diejenigen gebundenen Konturen, welche die ersten inselartigen Bereiche begrenzen und auch die gegebenenfalls zusätzlich vorhandenen gebundenen Teilbereiche zwischen den ersten inselartigen Bereichen verstanden.

Das den Hüftgürtel zumindest abschnittsweise bildende Vliesstoffmaterial kann bevorzugtermaßen ein Spinnvlies oder ein Kardenvlies oder ein Meltblownvlies oder ein wasservernadeltes Vlies umfassen. Es kann sich in vorteilhafter Weise auch um ein Vlieslaminat handeln, welches aus mehreren Vliesschichten besteht. Nach einer bevorzugten Ausführungsform der Erfindung umfasst das Material ein Spinnvlies und ein Kardenvlies, die miteinander verbunden sind. Solchenfalls bildet das Kardenvlies in bevorzugter Weise die erste Oberseite, welche eine Landezone für eine hakenbildende Komponente eines mechanischen Verschlussmittels bildet.

Wenn das Vliesstoffmaterial aus mehreren Vliesschichten besteht, es sich also um ein Vlieslaminat handelt, so erweist es sich als vorteilhaft, wenn diese Schichten durch Aufbringung der die ersten größeren ungebundenen inselartigen Bereiche begrenzenden gebundenen Konturen miteinander verbunden werden. Dies kann beispielsweise durch Thermoprägung, und zwar insbesondere durch Kalanderprägen oder Ultraschallschweißen erreicht werden. Bei der Ausbildung des Vliesstoffmaterials als Vliesstofflaminat kann, um die Festigkeit insgesamt zu erhöhen, eine von der ersten Oberseite abgewandte Vliesschicht durch ein weiteres, insbesondere durch Thermoprägung erzeugtes Bindemuster verfestigt sein. Diese weitere Vliesschicht bildet also nicht die Landezone für eine hakenbildende Komponente des Verschlussmittels, sondern sie ist auf der abgewandten Seite vorgesehen. Das Bindemuster wurde vorzugsweise bei der vorherigen Fertigung der weiteren Vliesschicht zu deren Vorverfestigung angebracht.

Das Flächengewicht dieses Vliesmaterials beträgt bevorzugtermaßen 15 bis 120 g/m², insbesondere von 20 bis 90 g/m², insbesondere von 30 bis 80 g/m², insbesondere von 40 bis 70 g/m² und weiter insbesondere von 50 bis 65 g/m ²

Das Flächengewicht derjenigen Vliesschicht, welche die erste Oberseite bildet und die ersten ungebundenen inselartigen Bereiche, die gebundenen Konturen und die zweiten ungebundenen Bereiche aufweist, beträgt vorzugsweise 10 bis 60 g/m², insbesondere von 10 bis 40 g/m², insbesondere von 15 bis 35 g/m² und weiter insbesondere von 20 bis 35 g/m².

Mit den vorerwähnten Flächengewichten in Verbindung mit der beanspruchten Ausgestaltung der ersten Oberseite lassen sich Vliesstoffmaterialien herstellen, die nicht nur eine gute Verschlussfunktion im Zusammenwirken mit einer hakenbildenden Komponente eines Verschlussmittels gewährleisten, sondern die zudem gute mechanische Eigenschaften insbesondere im Hinblick auf eine angenehme Biegsamkeit aufweisen und zudem auch tragende Funktion übernehmen können.

Im Hinblick auf die Ausbildung des Vliesstoffmaterials erweist es sich als vorteilhaft und zweckmäßig, wenn diejenige Vliesschicht, welche die erste Oberseite bildet, Fasern einer Stärke von 1 bis 10 dtex, vorzugsweise von 2 bis 8 dtex und weiter bevorzugtermaßen von 3 bis 6 dtex umfasst oder aus solchen Fasern besteht. Des Weiteren erweist es sich als vorteilhaft, wenn diejenige Vliesschicht, welche die erste Oberseite bildet, hydrophile Fasern umfasst oder aus hydrophilen Fasern besteht.

Wenn das Vliesstoffmaterial aus mehreren Vliesschichten besteht, so kann es eine Vliesschicht als Träger umfassen, die ein Flächengewicht von 10 bis 100 g/m², vorzugsweise von 15 bis 60 g/m² und weiter vorzugsweise von 30 bis 40 g/m² aufweist. Diese Trägerschicht ist dann von der ersten Oberseite abgewandt angeordnet. Sie umfasst vorzugsweise Fasern einer Stärke von 1 bis 6 dtex, vorzugsweise von 1 bis 4 dtex und weiter vorzugsweise von 2 bis 4 dtex.

In besonders vorteilhafter Weiterbildung der Erfindung ist das Vliesstoffmaterial atmungsaktiv, das heißt es lässt Wasser passieren, und /oder luftdurchlässig. Hierdurch wird der Tragekomfort verbessert.

Wie bereits angedeutet, erweist es sich als vorteilhaft, wenn das Vliesstoffmaterial eine Steifigkeit von < 0,80 N, insbesondere von < 0,60 N, insbesondere von < 0,40 N, insbesondere von < 0,30 N, insbesondere von < 0,25 N, insbesondere von < 0,2 N, insbesondere < 0,18 N, und weiter insbesondere < 0,16 N, jedoch von wenigstens 0,05 N aufweist. Diese Steifigkeit wird bestimmt nach der in EP 0 699 066 B1 beschriebenen Prüfmethode der modifizierten Version des Tests ASTM D 4032-82 CIRCULAR BEND, so dass zu Zwecken der Offenbarung hierauf ausdrücklich Bezug genommen wird und der diesbezügliche Inhalt von EP 0 699 066 B1 in die vorliegende Anmeldung mit einbezogen wird. Ergänzend hierzu wird diese Prüfmethode dahingehend weiter spezifiziert, dass bei der Prüfanordnung die erste Oberseite des Vliesstofflaminates unten zu liegen kommt, das heißt diejenige Oberfläche bildet, die dem Stempel der Prüfapparatur abgewandt ist.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Windel. In der Zeichnung zeigt:
- Figur 1: eine schematische Darstellung einer erfindungsgemäßen Gürtelwindel mit geöffnetem Hüftgürtel;
- Figur 2: eine schematische Schnittansicht der ersten Verschlussmittel mit Schnittebene A-A der Gürtelwindel nach Figur 1
- Figur 3: eine schematische Darstellung einer erfindungsgemäßen Gürtelwindel nach Figur 1 mit geschlossenem Hüftgürtel;
- Figuren 4 und 5: Darstellungen einer hakenbildenden Komponente eines Verschlussmittels.
- Figur 6: eine schematische Schnittansicht des Vliesstoffmaterials aus Figur 7a;
- Figur 7a: eine Draufsicht auf die Abwicklung einer Gravurwalze zum Prägen eines Vliesstoffmaterials; außerdem gleichzeitig schematisch eine Ansicht der ersten Oberseite des die Außenseite des Hüftgürtels bildenden Vliestoffmaterials;
- Figur 7b: eine Schnittansicht (Detail) aus Figur 7a;
- Figur 8: eine schematische Darstellung einer erfindungsgemäßen Gürtelwindel mit gefaltetem Hüftgürtel;
- Figur 9: eine schematische Schnittansicht mit Schnittebene A-A der Gürtelwindel nach Figur 8 mit einer ersten Faltung des Hüftgürtels;
- Figur 10: eine schematische Darstellung des Aufbaus eines Zugprüfversuchs mit einer Vorrichtung mit gekrümmter Fläche;
- Figur 11a, 11b: schematische Darstellungen des Prüflings;
- Figur 12: eine perspektivische Ansicht der in Figur 10 dargestellten Vorrichtung

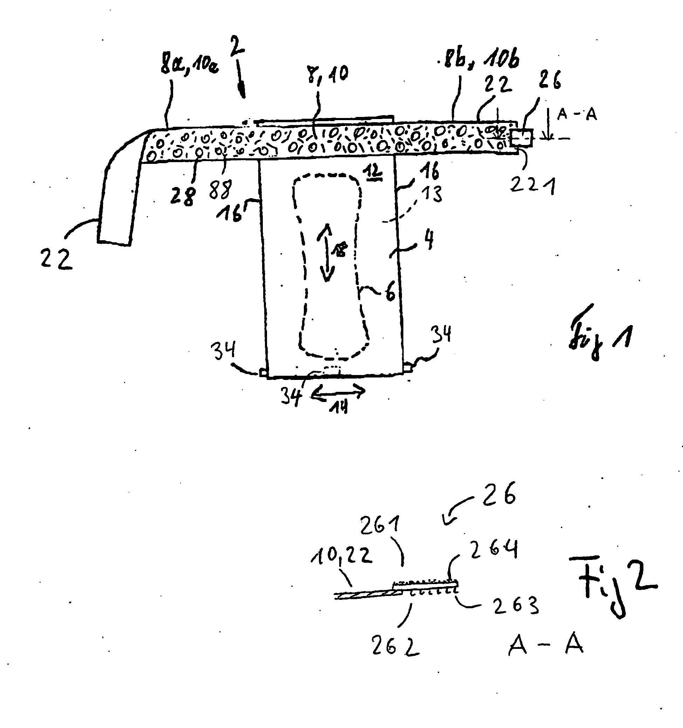

Figur 1 zeigt schematisch eine wegwerfbare Gürtelwindel 2 mit einem Hauptteil 4 und einem angedeuteten Absorptionskörper 6. An den Hauptteil 4 angefügt ist ein einstückiger Materialabschnitt 8, welcher einen Hüftgürtel 10 der Gürtelwindel bildet. Figur 1 zeigt den Gürtel in entfaltetem Zustand. Der einstückige Materialabschnitt 8 ist an eine Außenseite 12 des Hauptteils 4 unlösbar angefügt. Er erstreckt sich in Querrichtung 14 der Gürtelwindel 2 über seitliche Längsränder 16 im entfalteten Zustand jeweils über wenigstens 300 mm, insbesondere über wenigstens 400 mm, insbesondere über wenigstens 500 mm, insbesondere über wenigstens 600 mm, insbesondere über wenigstens 700 mm hinaus. Im dargestellten Fall der Figur 1 ist der erste Materialabschnitt 8a deutlich länger als der zweite Materialabschnitt 8b, das heißt die Erstreckung des ersten Hüftgürtelabschnittes 10a im entfalteten Zustand in Querrichtung 14 über den Längsrand 16 des Hauptteils 4 hinaus ist größer, vorzugsweise um 100mm größer, besonders bevorzugt um 200 mm größer ganz besonders bevorzugt um 300mm größer als die des zweiten Hüftgürtelabschnittes 10b.

Es wird aber darauf hingewiesen, dass erster und zweiter Materialabschnitt auch gleich lang sein können.

Am zweiten Hüftgürtelabschnitt 10b ist an dessen Ende auch ein erstes Verschlussmittel 26 vorgesehen, und zwar in Form einer mechanische Verschlusselemente aufweisenden Lasche 26, die über den Seitenrand 221 des zweiten Hüftgürtelabschnittes 10b übersteht, und zwar vorzugsweise um mindestens 10 mm, insbesondere um mindestens 20 mm, weiter insbesondere um mindestens 25 mm. Aus Figur 2 ist erkennbar, dass das erste Verschlussmittel 26 eine körperzugewandte Seite 262 und eine körperabgewandte Seite 261 aufweist. Die körperzugewandte Seite 262 umfasst mechanische Verschlusselemente, nämlich Kletthaken 263, welche mit Gegenverschlusselementen, nämlich zweiten Verschlusselementen 88 am ersten Hüftgürtelabschnitt 10a, insbesondere mit dessen gesamter Außenfläche lösbar haftend zusammenwirken kann, wenn der Hüftgürtel 10 zur Bildung einer in Hüftumfangsrichtung geschlossenen Hüftöffnung geschlossen wird.
Die körperabgewandte Seite 261 umfasst ein Vliesmaterial 264, das in unten noch näher beschriebener Weise mit den Kletthaken 341 aufweisenden mechanischen Verschlussmitteln 34 haftend zusammenwirken kann. Diese Verschlussmittel 34 werden im dargestellten Fall durch das Material "Microplast" 42-288-HX200-PP3 von der Firma G. Binder GmbH & Co. KG Textil- und Kunststofftechnik, Holzgerlingen, gebildet. Darstellungen der Form der hakenbildenden Komponente sind in den Figuren 4 und 5 dargestellt. Die Haken haben eine pilzförmige Gestalt mit ungefähr hexagonaler Fläche des Kopfs. Es befinden sich etwa 288 solcher pilzförmiger Vorsprünge pro cm². Das Material besteht aus Polypropylen und weist eine Dicke von ca. 0,42 mm auf. Die Höhe der pilzförmigen Erhebung gegenüber dem Grund des Materials beträgt ca. 0,26 mm. Der Abstand der Kanten der Köpfe beträgt ca. 200 µm.

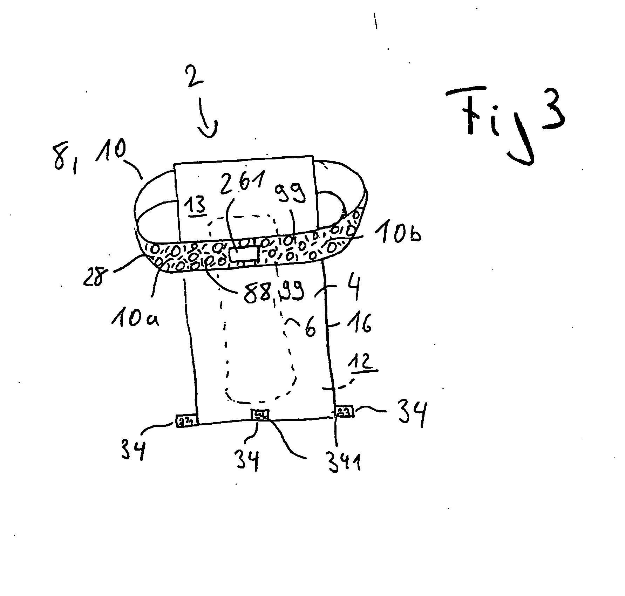

Figur 3 zeigt eine Ansicht der Innenseite 13 der Gürtelwindel 2 nachdem der erste Hüftgürtelabschnitt 10b in überlappender Konfiguration an den zweiten Verschlusselementen 88 des ersten Hüftgürtelabschnittes zur Bildung der geschlossenen Hüftöffnung festgelegt wurde.

Zum endgültigen Anlegen der Gürtelwindel 2 muss nun der Hauptteil 4 zwischen den Beinen eines Benutzers hervorgeholt und über weitere mechanische Verschlusselemente insbesondere Kletthaken 341 aufweisende Laschen 34 des Hauptteils 4 an der Außenseite des Hüftgürtels 10 lösbar festgelegt werden. Erfindungsgemäß stehen hierfür nicht nur die vierten Verschlussmittel 99 auf der Außenseite beider Hüftgürtelabschnitte zur Verfügung, sondern außerdem die aus einem Vliesstoffmaterial 264 gebildete körperabgewandte Seite 261 des ersten Verschlussmittels 26.

Im dargestellten Fall sind die in Figur 2 schematisch dargestellten ersten Verschlussmittel 26 aus einem bei der Fa. Köster GmbH & Co KG, ansässig in Altendorf, Bundesrepublik Deutschland, unter der Bezeichnung CP2 EM 67 precut HC erhältlichen elastischen Klettwindeltape gebildet. Die Außenseite dieses Tapes, somit die körperabgewandte Seite des Verschlussmittels 26, wird vollflächig von dem Vliesstoffmaterial 264 abgedeckt. Das Vliesstoffmaterial 264 besteht aus einem thermisch rautenpunktgeprägtem PP-Spinnvlies, auf dessen Außenseite ein Film eines PP/PE-Polymers direkt extrudiert ist, um das auf der Unterseite zur Fixierung am Seitenrand 221 des zweiten Hüftgürtelabschnittes 10b mit einem Haftkleber versehene Klettwindeltape tapeherstellerseitig endlos auf sich selbst ohne weitere Trennmaterialien wie Release-Papiere aufrollen und windelherstellerseitig mühelos abrollen zu können. Das Flächengewicht des mit dem PP/PE-Polymer beschichteten Vliesstoffmaterials beträgt 75 g/m².

Die nach oben beschriebener Prüfmethode ermittelte Scherkraft zwischen der körperabgewandten Seite 261 des ersten Verschlussmittels 26 und den dritten Verschlussmitteln 34 beträgt 7,97 N/25mm.

Diese Kraft reicht unter normalen Tragebedingungen aus, um einen sicheren Verschluss der Windel in Gebrauch zu gewährleisten.

Die Gürtelwindel kann hierdurch überaus flexibel an unterschiedliche Anatomien bzw. Konfektionsgrößen angepasst werden.

Im dargestellten Fall einer bevorzugten Ausführungsform umfasst die gesamte Außenseite, das heißt die körperabgewandte Seite des Hüftgürtels 10 ein schlaufenbildendes Vliesstoffmaterial, welches zugleich sowohl die zweiten Verschlusselementen 88 als auch die vierten Verschlusselemente 99 bildet. Dieses schlaufenbildende Vliesstoffmaterial ist mit Bezugszeichen 28 angedeutet.

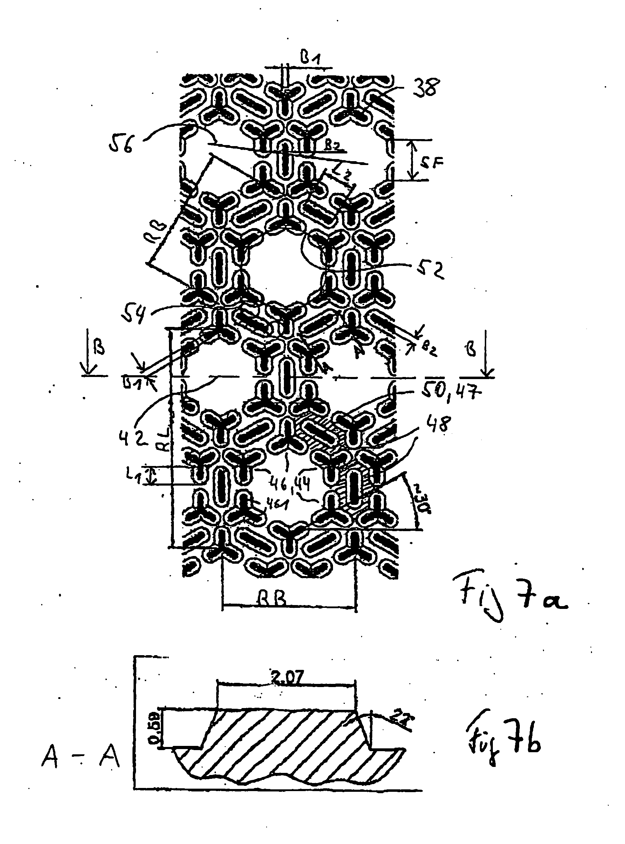

Das Vliesstoffmaterial 28 besteht aus einem Vliesstofflaminat 30, das im Schnitt in Figur 6 schematisch dargestellt ist. Die Schnittlinie verläuft dabei entlang einer aus Figur 7a ersichtlichen Linie B-B. Es weist eine Polypropylen - Spinnvliesschicht 32 bestehend aus Fasern einer Faserstärke von 2,2 dtex mit einem Flächengewicht von 30 g/m² als Träger auf, die durch ovale Bindepunkte 31 einer Flächendichte von 48,37 Stück/cm² (thermische Prägepunkte) vorverfestigt wurde, wobei die Abmessungen der Halbachsen der ovalen Bindepunkte jeweils 0,85 mm und 0,59 mm betragen und die Pressfläche der ovalen Bindepunkte dementsprechend 0,394 mm² und der Anteil der Bindepunkte an der Gesamtfläche somit 19,0 % beträgt. Die Tiefe der Bindepunkte beträgt 0,80 mm. Auf diese Spinnvliesschicht 32 ist eine Kardenvliesschicht 36 angefügt, und zwar durch eine Heißkalanderprägung. Im dargestellten Fall besteht das Kardenvlies aus hydrophilisierten Polypropylenfasern einer Stärke von 4,4 dtex und einer Faserlänge von 40 mm. Figur 7a zeigt eine Ansicht der Abwicklung der Oberfläche einer Kalanderwalze, welche einem durch Heißkalandern auf die erste Oberseite 40 der Kardenvliesschicht 36 aufgebrachten Prägemuster 38 entspricht Figur 7a zeigt schematisch somit gleichzeitig auch eine Ansicht der ersten Oberseite 40 des Vliesstofflaminats 30.

Durch Aufbringung des Prägemusters 38 werden auf der ersten Oberseite 40 des Vliesstofflaminats 30 erste größere ungebundene Bereiche 42 gebildet, die begrenzt werden durch eine thermisch gebundene Kontur 44 aus unterbrochenen ersten Segmenten 46. Diese ersten Segmente 46 weisen im dargestellten Fall eine in der Aufsicht regelmäßige trilobale Form auf. Die Arme 461 dieser ersten Segmente 46 weisen eine Länge L1 von 1,04 mm und eine Breite B1 von 0,47 mm auf. Die so begrenzten ersten ungebundenen Bereiche sind sechseckförmig und inselartig auf der ersten Oberseite 40 angeordnet und voneinander beabstandet. Zwischen den ersten Bereichen 42 befinden sich zweite ungebundene Bereiche 48, die kleiner als die ersten ungebundenen Bereiche 42 sind. Diese zweiten ungebundenen Bereiche umschließen wiederum gebundene Teilbereiche 50, die stegartig durch zweite Segmente 47 gebildet sind. Diese zweiten Segmente 47 weisen eine Länge L2 von 2,07 mm und eine Breite B2 von 0,47 mm auf. Die Prägetiefe der ersten und zweiten Segmente beträgt jeweils 0,59 mm. Im beispielhaft dargestellten Fall haben die ersten ungebundenen inselartigen Bereiche eine Abmessung der Seiten der Sechseckform SF von 2,9 mm. Ihr Abstand beträgt ca. 2,6 mm.

Es ist insbesondere nicht möglich, einen in die ersten größeren Bereiche 42 einbeschriebenen größtmöglichen Kreis 52, der im dargestellten Fall einen Durchmesser von 4,8 mm besitzt, innerhalb der zweiten ungebundenen kleineren Bereiche 48 anzuordnen. Dort sind größtmögliche Kreise 54 angedeutet.

Die Rapportbreite (RB) des Prägemusters 38 beträgt 8 mm, die Rapportlänge RL beträgt 13,86 mm.

Der gesamte Flächenanteil gebundener Bereiche 44, 50 beträgt 20,7 %. Der Flächenanteil der ersten größeren ungebundenen inselartigen Bereiche (42) beträgt ca. 35,7 %, wobei zur Ermittlung der Fläche eines ersten ungebundenen inselartigen Bereiches die Größe der Fläche des eingeschriebenen Kreises 52 herangezogen wird.

Die nach oben beschriebener Prüfmethode ermittelte Scherkraft dieses Vliesstoffmaterials beträgt 51,00 N/25 mm, wobei als Kletthakenkomponente das oben näher gekennzeichnete Material "Microplast" 42-288-HX200-PP3 von der Firma G. Binder GmbH & Co. KG Textil- und Kunststofftechnik, Holzgerlingen, verwendet wurde.

Die Steifigkeit des Vliesstoffmaterials ermittelt nach der in EP0699066B1 offenbarten Prüfmethode beträgt 0,13 N, wobei bei der Prüfanordnung die Spinnvliesseite oben zu liegen kommt, das heißt diejenige Oberfläche bildet, die dem Stempel der Prüfapparatur zugewandt ist.

Bei einem weiteren Ausführungsbeispiel der vorliegenden Erfindung wurde lediglich das Flächengewicht des Spinnvlieses auf 45 g/m² erhöht und das die Vliesstoffe verbindende Prägemuster 38 wie folgt modifiziert:
Länge L1 der Arme 461 der ersten Segmente 46: 1,0 mm
Breite B1 der Arme 461 der ersten Segmente 46: 0,4 mm
Länge L2 der zweiten Segmente 47: 2,0 mm
Breite B2 der zweiten Segmente 47: 0,4 mm
Flächenanteil gebundener Bereiche: 17,1 %
Prägetiefe der ersten und zweiten Segmente: 0,68 mm

Die nach oben beschriebener Prüfmethode ermittelte Scherkraft dieses weiteren Ausführungsbeispiels beträgt 58,89 N/25 mm, wobei als Kletthakenkomponente wiederum das oben näher gekennzeichnete Material "Microplast" 42-288-HX200-PP3 von der Firma G. Binder GmbH & Co. KG Textil- und Kunststofftechnik, Holzgerlingen, verwendet wurde.

Die Steifigkeit dieses weiteren Vliesstoffmaterials ermittelt nach der in EP0699066B1 offenbarten Prüfmethode beträgt 0,28 N, wobei bei der Prüfanordnung die Spinnvliesseite oben zu liegen kommt, das heißt diejenige Oberfläche bildet, die dem Stempel der Prüfapparatur zugewandt ist.

Jede gerade Verbindungslinie 56 zwischen benachbarten ersten größeren Bereichen 42 verläuft stets durch eine gebundene Kontur 44 oder einen gebundenen Teilbereich 50, der zwischen den ersten größeren Bereichen 42 angeordnet ist.

Bevorzugt und beispielhaft dargestellte Abmessungen der Prägestruktur 38 sind in den Figuren 7a und 7b dargestellt, wobei Figur 7b einen Schnitt entlang der Linie A-A durch ein zweites Segment 56 darstellt.

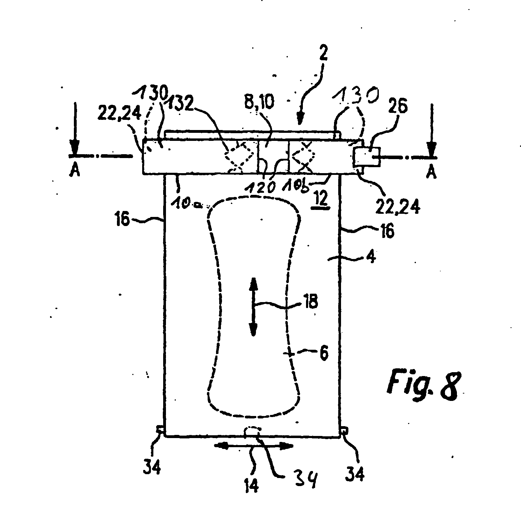

Figur 8 zeigt schematisch die wegwerfbare Gürtelwindel 2 mit beidseits einer Windellängsrichtung 18 um Falzlinien 120 mehrfach auf sich selbst gefaltetem den Hüftgürtel 10 bildenden Materialabschnitt 8. Das jeweils freie Ende 22 des Materialabschnitts 8 bildet einen über die Längsseitenränder 16 um wenigstens 10 mm vorstehenden Anfassbereich 24 zum Ergreifen mit den Fingern eines Benutzers. Dort ist auch das Verschlusselement 26 an einem freien Ende 22 vorgesehen.

Die aufeinander gefalteten Teilabschnitte 130 des einstückigen Materialabschnitts 8 sind lösbar aufeinander fixiert, und zwar durch eine Anzahl von im Wesentlichen punktförmigen Fügestellen 132 in Form von vorzugsweise Ultraschallschweißpunkten. Hierfür sind einige wenige Fügestellen ausreichend.

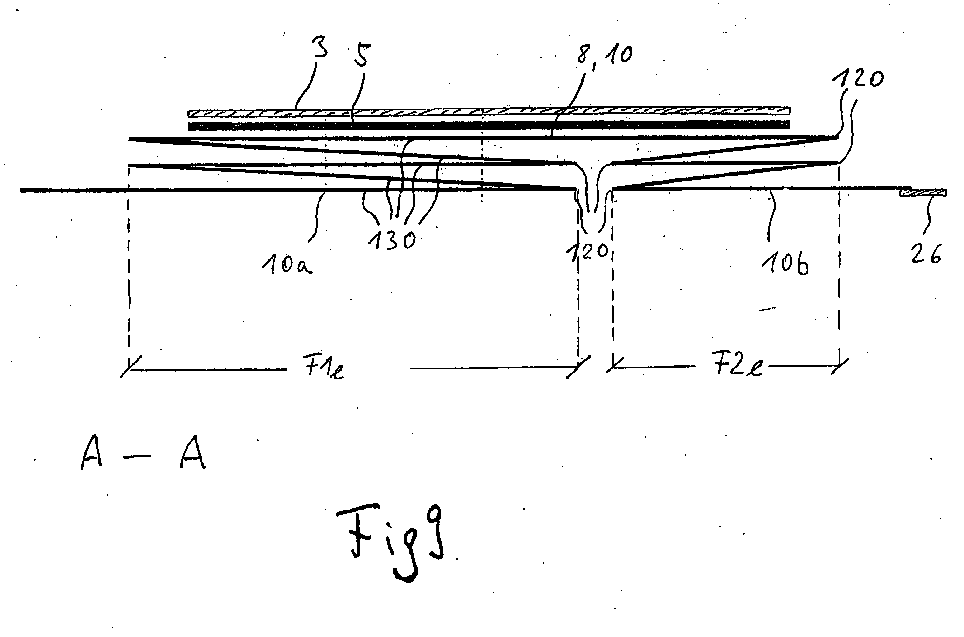

Die Figur 9 als Schnittansicht entlang der Linie A-A in Figur 8 zeigt eine besonders bevorzugte Faltung des einstückigen Materialabschnitts 8, der außen, also körperabgewandt, am Ende des Windelhauptteils 4 angebracht ist. Der Windelhauptteil wird an seinem Ende im dargestellten Fall durch ein körperseitiges Topsheet 3 und ein wäscheseitiges Backsheet 5 gebildet. Die Faltung umfasst auf jeder Seite vier Falzlinien 120; eine jeweilige Faltung ist also doppel-Z-förmig. Der Gürtel ist asymmetrisch gefaltet. Die Faltbreite (F1,1) des ersten längeren Hüftgürtelabschnitts 10a ist größer als die Faltbreite (F2,1) des kürzeren zweiten Hüftgürtelabschnitts 10b.

Es wir darauf hingewiesen, dass der Gürtel 10 natürlich auch symmetrisch gefaltet werden kann, also mit im Wesentlichen identischen Faltbreiten (F1,1; F2,1), insbesondere dann, wenn erster und zweiter Hüftgürtelabschnitt (10a, 10b) eine im Wesentlichen gleiche Länge aufweisen.

Die aufeinander gefalteten Teilabschnitte 130 jedes Hüftgürtelabschnittes 10a, 10b sind lösbar aufeinander fixiert, und zwar durch eine Anzahl von im Wesentlichen punktförmigen Fügestellen 132 in Form von vorzugsweise Ultraschallschweißpunkten (in Figur 9 nicht dargestellt).

## Patentansprüche

1. Wegwerfwindel (2), insbesondere zur Inkontinentenversorgung, mit einem Hüftgürtel (10), wobei der Hüftgürtel (10) einen ersten Hüftgürtelabschnitt (10a) und einen zweiten Hüftgürtelabschnitt (10b) aufweist und wobei zumindest der zweite Hüftgürtelabschnitt (10b) ein erstes Verschlussmittel (26) aufweist in Form einer mechanische Verschlusselemente aufweisenden Lasche (26), die über einen Seitenrand (221) des zweiten Hüftgürtelabschnitts (10b) übersteht, mit einer im Gebrauchszustand körperabgewandten Seite (261) und einer im Gebrauchszustand körperzugewandten Seite (262), und zumindest der erste Hüftgürtelabschnitt (10a) ein zweites Verschlussmittel (88) aufweist, wobei die körperzugewandte Seite (262) des ersten Verschlussmittels (26) an dem zweiten Verschlussmittel (88) zur Bildung einer geschlossenen Hüftöffnung lösbar festlegbar ist, und mit einem einen Vorderbereich, einen Rückenbereich und einen dazwischenliegenden Schrittbereich aufweisenden Windelhauptteil (4), der einen Absorptionskörper (6) für Flüssigkeiten aufweist, wobei der Windelhauptteil (4) mit dem Längsende seines Vorderbereichs oder seines Rückenbereichs über mindestens ein drittes, mechanisches Verschlussmittel (34) lösbar an einem vierten Verschlussmittel (99) auf der körperabgewandten Seite des Hüftgürtels (10) festlegbar ist, **dadurch gekennzeichnet, dass** das dritte mechanische Verschlussmittel (34) außerdem an der körperabgewandten Seite (261) des ersten Verschlussmittels (26) lösbar festlegbar ist.

2. Wegwerfwindel nach Anspruch 1, **dadurch gekennzeichnet dass** die körperabgewandte Seite (261) des ersten Verschlussmittels (26) zumindest abschnittsweise durch ein Fasermaterial (264) gebildet ist, an das die dritten Verschlussmittel (34) lösbar festlegbar sind.

3. Wegwerfwindel nach einem der vorgenannten Ansprüche, **dadurch gekennzeichnet, dass** das dritte Verschlussmittel (34) Kletthaken umfasst.

4. Wegwerfwindel nach einem der vorgenannten Ansprüche, **dadurch gekennzeichnet, dass** das erste Verschlussmittel (26) zumindest abschnittsweise elastisch ist.

5. Wegwerfwindel nach einem der vorgenannten Ansprüche, **dadurch gekennzeichnet, dass** das erste Verschlussmittel (26) mechanische Verschlusselemente, insbesondere Kletthaken (263) umfasst.

6. Wegwerfwindel nach einem der vorgenannten Ansprüche, **dadurch gekennzeichnet, dass** das erste Verschlussmittel (26) ein elastisches Vliesmaterial umfasst, an das das dritte Verschlussmittel (34) lösbar festlegbar ist.

7. Wegwerfwindel nach einem der vorgenannten Ansprüche, **dadurch gekennzeichnet, dass** die Haftkraft als Scherkraft zwischen der körperabgewandten Seite (261) des ersten Verschlussmittels (26) und dem drittem Verschlussmittel (34) geringer ist als die Haftkraft als Scherkraft zwischen der körperzugewandten Seite (262) des ersten Verschlussmittels (26) und dem zweiten Verschlussmittel (88) .

8. Wegwerfwindel nach einem der vorgenannten Ansprüche, **dadurch gekennzeichnet dass** die Haftkraft als Scherkraft zwischen der körperzugewandten Seite (262) des ersten Verschlussmittels (26) und dem zweiten Verschlussmittel (88) wenigstens 5 N/25mm, insbesondere wenigstens 10N/25mm, weiter insbesondere wenigstens 15N/25mm, weiter insbesondere wenigstens 20N/25mm, weiter insbesondere wenigstens 25N/25mm, weiter insbesondere wenigstens 30N/25mm, weiter insbesondere wenigstens 35N/25mm, weiter insbesondere wenigstens 40N/25mm, weiter insbesondere wenigstens 45N/25mm und weiter insbesondere wenigstens 50N/25mm beträgt, jedoch insbesondere höchstens 80N/25mm, und weiter insbesondere höchstens 70N/25mm beträgt.

9. Wegwerfwindel nach einem der vorgenannten Ansprüche, **dadurch gekennzeichnet dass** die Haftkraft als Scherkraft zwischen der körperabgewandten Seite (261) des ersten Verschlussmittels (26) und dem dritten Verschlussmittel (34) wenigstens 2 N/25mm, insbesondere wenigstens 3,5 N/25mm, weiter insbesondere wenigstens 5N/25mm, weiter insbesondere wenigstens 10N/25mm, weiter insbesondere wenigstens 15N/25mm, weiter insbesondere wenigstens 20N/25mm, weiter insbesondere wenigstens 25N/25mm, weiter insbesondere wenigstens 30N/25mm, weiter insbesondere wenigstens 35N/25mm, weiter insbesondere wenigstens 40N/25mm, weiter insbesondere wenigstens 45N/25mm und weiter insbesondere wenigstens 50N/25mm beträgt, jedoch insbesondere höchstens 70N/25mm, und weiter insbesondere höchstens 60N/25mm beträgt

10. Wegwerfwindel nach einem oder mehreren der vorstehenden Ansprüche, **dadurch gekennzeichnet, dass** der Hüftgürtel (10) von einem einstückigen Materialabschnitt (8) gebildet ist, der an den Windelhauptteil (4) angefügt ist.

11. Wegwerfwindel nach Anspruch 10 **dadurch gekennzeichnet, dass** der einstückige Materialabschnitt, der an den Windelhauptteil (4) angefügt ist, beidseits einer Längsmittelachse um in einer Längsrichtung (18) der Windel erstreckte Falzlinien (120) auf sich selbst gefaltet ist.

12. Wegwerfwindel nach einem oder mehreren der vorstehenden Ansprüche, **dadurch gekennzeichnet, dass** der Hüftgürtel (10) in gefalteter Konfiguration mit einem Anfassbereich (24) an seinem freien Ende (22) über einen Längsseitenrand (16) des Windelhauptteils (4) in Querrichtung (14) um insbesondere wenigstens 10 mm, insbesondere wenigstens 20 mm, weiter insbesondere wenigstens 30 mm, weiter insbesondere wenigstens 40 mm, weiter insbesondere wenigstens 50 mm und weiter insbesondere wenigstens 60 mm vorsteht.

13. Wegwerfwindel nach einem oder mehreren der vorstehenden Ansprüche, **dadurch gekennzeichnet, dass** der Hüftgürtel (10)in gefalteter Konfiguration mit einem Anfassbereich (24) an seinen beiden freien Enden (22) über einen Längsseitenrand (16) des Windelhauptteils (4) in Querrichtung (14) vorsteht.

14. Wegwerfwindel nach einem oder mehreren der vorstehenden Ansprüche, **dadurch gekennzeichnet, dass** die Erstreckung des den Hüftgürtel (10) bildenden Materialabschnitts im entfalteten Zustand in Querrichtung (14) über den Längsrand (16) des Hauptteils (4) hinaus wenigstens 200 mm, insbesondere wenigstens 300 mm, insbesondere wenigstens 400 mm, insbesondere wenigstens 500 mm und weiter insbesondere wenigstens 600 mm beträgt.

15. Wegwerfwindel nach einem oder mehreren der vorstehenden Ansprüche, **dadurch gekennzeichnet, dass** der Hüftgürtel (10) eine Erstreckung in Längsrichtung (18) von 30 bis 150 mm, insbesondere von 40 bis 130 mm, insbesondere von 50 bis 125 mm, insbesondere von 60 bis 120 mm, insbesondere von 70 bis 115 mm und weiter insbesondere von 75 bis 110 mm aufweist.

16. Wegwerfwindel nach einem oder mehreren der vorstehenden Ansprüche, **dadurch gekennzeichnet, dass** der Hüftgürtel (10) unlösbar an eine Außenseite (12) des Hauptteils (4) angefügt ist.

17. Wegwerfwindel nach einem der vorgenannten Ansprüche, **dadurch gekennzeichnet, dass** das zweite Verschlussmittel (88) und / oder das vierte Verschlussmittel (99) ein schlaufenbildendes Vliesstoffmaterial (30) als mechanisches Verschlussmittel umfasst, wobei eine erste, in Gebrauch körperabgewandt angeordnete Oberseite (40) des Vliesstoffmaterials (30) erste größere ungebundene Bereiche (42) umfasst, die voneinander beabstandet inselartig angeordnet sind, und die ersten größeren ungebundenen Bereiche (42) von gebundenen Konturen (44) begrenzt sind und außerhalb der Begrenzung von zweiten kleineren ungebundenen Bereichen (48) umgeben und durch diese zweiten kleineren ungebundenen Bereiche (48) voneinander beabstandet sind.

18. Wegwerfwindel nach Anspruch 17, **dadurch gekennzeichnet, dass** die zweiten kleineren ungebundenen Bereiche (48) ihrerseits gebundene Teilbereiche (50) umschließen können.

19. Wegwerfwindel nach Anspruch 17 oder nachfolgend, **dadurch gekennzeichnet, dass** jede gerade Verbindungslinie (56) zwischen benachbarten ersten größeren Bereichen (42) stets durch eine gebundene Kontur (44) oder einen gebundenen Teilbereich (50), der zwischen den ersten größeren Bereichen (42) angeordnet ist, verläuft.

20. Wegwerfwindel nach Anspruch 17 oder nachfolgend, **dadurch gekennzeichnet, dass** die ersten größeren ungebundenen inselartigen Bereiche (42) eine Abmessung, insbesondere einen Durchmesser eines in den ersten Bereich einbeschriebenen Kreises (52), von 2 - 15 mm, insbesondere von 3 - 10 mm und weiter insbesondere von 3 - 8 mm aufweisen.

21. Wegwerfwindel nach Anspruch 17 oder nachfolgend, **dadurch gekennzeichnet, dass** die ersten größeren ungebundenen inselartigen Bereiche (42) einen Flächenanteil von 5 - 75 %, insbesondere von 5 - 60 %, insbesondere von 10 - 50 %, insbesondere von 10 - 45 %, insbesondere von 15 - 45 %, insbesondere von 20 - 40 % und weiter insbesondere von 30 - 40 % der Gesamtfläche der ersten Oberseite aufweist.

22. Wegwerfwindel nach Anspruch 17 oder nachfolgend **dadurch gekennzeichnet, dass** die ersten größeren ungebundenen inselartigen Bereiche (42) kreisförmig oder oval oder dreieckig oder vieleckig, insbesondere sechseckig, ausgebildet sind.

23. Wegwerfwindel nach Anspruch 17 oder nachfolgend **dadurch gekennzeichnet, dass** die ersten größeren ungebundenen inselartigen Bereiche (42) wenigstens 1 mm, insbesondere wenigstens 1,5 mm, insbesondere wenigstens 2 mm, insbesondere wenigstens 2,5 mm und weiter insbesondere wenigstens 3 mm voneinander beabstandet sind.

24. Wegwerfwindel nach Anspruch 17 oder nachfolgend, **dadurch gekennzeichnet, dass** der gesamte Flächenanteil gebundener Bereiche 10 -60 %, insbesondere 15 - 40 %, insbesondere 15 - 30 %, insbesondere 15 - 25 %, insbesondere 19 - 22 % der Gesamtfläche der ersten Oberfläche beträgt.

25. Wegwerfwindel nach Anspruch 17 oder nachfolgend, **dadurch gekennzeichnet, dass** das schlaufenbildende Vliesstoffmaterial eine Steifigkeit von höchstens 0,80 N, insbesondere von höchstens 0,60 N, insbesondere von höchstens 0,40 N, insbesondere von höchstens 0,30 N, insbesondere von höchstens 0,25 N, insbesondere von höchstens 0,20 N, insbesondere von höchstens 0,18 N, und weiter insbesondere von höchstens 0,16 N, jedoch von wenigstens von 0,05 N aufweist.

## Claims

1. Disposable nappy (2), in particular for incontinence care, comprising a hip belt (10), the hip belt (10) having a first hip belt portion (10a) and a second hip belt portion (10b) and at least the second hip belt portion (10b) having a first closing means (26) in the form of a strap (26) which comprises mechanical closing elements and projects over a side edge (221) of the second hip belt portion (10b), having a side (261) facing away from the user during use and a side (262) facing the user during use, and at least the first hip belt portion (10a) having a second closing means (88), the user-facing side (262) of the first closing means (26) being detachably fixable to the second closing means (88) to form a closed hip opening, and comprising a nappy main part (4) having a front region, a rear region, an intermediate crotch region, and an absorptive element (6) for liquids, the longitudinal end of the front region or rear region of the nappy main part (4) being detachably fixable, via at least a third mechanical closing means (34), to a fourth closing means (99) on the side of the hip belt (10) facing away from the user, **characterised in that** the third mechanical closing means (34) is also detachably fixable to the side (261) of the first closing means (26) facing away from the user.

2. Disposable nappy according to claim 1, **characterised in that** the side (261) of the first closing means (26) facing away from the user is formed, at least in portions, of a fibre material (264) to which the third closing means (34) is detachably fixable.

3. Disposable nappy according to any one of the preceding claims, **characterised in that** the third closing means (34) comprises Velcro-type hooks.

4. Disposable nappy according to any one of the preceding claims, **characterised in that** the first closing means (26) is resilient at least in portions.

5. Disposable nappy according to any one of the preceding claims, **characterised in that** the first closing means (26) comprises mechanical closing elements, in particular Velcro-type hooks (263).

6. Disposable nappy according to any one of the preceding claims, **characterised in that** the first closing means (26) comprises a resilient non-woven material to which the third closing means (34) is detachably fixable.

7. Disposable nappy according to any one of the preceding claims, **characterised in that** the adhesive force as a shearing force between the side (261) of the first closing means (26) facing away from the user and the third closing means (34) is less than the adhesive force as a shearing force between the side (262) of the first closing means (26) facing the user and the second closing means (88).

8. Disposable nappy according to any one of the preceding claims, **characterised in that** the adhesive force as a shearing force between the side (262) of the first closing means (26) facing the user and the second closing means (88) is at least 5 N/25 mm, in particular at least 10 N/25 mm, more particularly at least 15 N/25 mm, more particularly at least 20 N/25 mm, more particularly at least 25 N/25 mm, more particularly at least 30 N/25 mm, more particularly at least 35 N/25 mm, more particularly at least 40 N/25 mm, more particularly at least 45 N/25 mm, and more particularly at least 50 N/25 mm, but in particular at most 80 N/25 mm and more particularly at most 70 N/25 mm.

9. Disposable nappy according to any one of the preceding claims, **characterised in that** the adhesive force as a shearing force between the side (261) of the first closing means (26) facing away from the user and the third closing means (34) is at least 2 N/25 mm, in particular at least 3.5 N/25 mm, more particularly at least 5 N/25 mm, more particularly at least 10 N/25 mm, more particularly at least 15 N/25 mm, more particularly at least 20 N/25 mm, more particularly at least 25 N/25 mm, more particularly at least 30 N/25 mm, more particularly at least 35 N/25 mm, more particularly at least 40 N/25 mm, more particularly at least 45 N/25 mm and more particularly at least 50 N/25 mm, but in particular at most 70 N/25 mm and more particularly at most 60 N/25 mm.

10. Disposable nappy according to one or more of the preceding claims, **characterised in that** the hip belt (10) is formed by a single-piece material portion (8) which is joined to the nappy main part (4).

11. Disposable nappy according to claim 10, **characterised in that** the single-piece material portion which is joined to the nappy main part (4) is folded onto itself on both sides of a longitudinal central axis about fold lines (120) extending in a longitudinal direction (18) of the nappy.

12. Disposable nappy according to one or more of the preceding claims, **characterised in that** in a folded state of the hip belt, a grip region (24) at a free end (22) of said hip belt (10) projects in a transverse direction (14) past a longitudinal side edge (16) of the nappy main part (4) by in particular at least 10 mm, in particular at least 20 mm, more particularly at least 30 mm, more particularly at least 40 mm, more particularly at least 50 mm and more particularly at least 60 mm.

13. Disposable nappy according to one or more of the preceding claims, **characterised in that** in a folded state of the hip belt, a grip region (24) at both free ends (22) of said hip belt (10) projects in a transverse direction (14), past a longitudinal side edge (16) of the nappy main part (4).

14. Disposable nappy according to one or more of the preceding claims, **characterised in that** in an unfolded state of the hip belt, the material portion forming said hip belt (10) extends in a transverse direction (14) past the longitudinal edge (16) of the main part (4) by at least 200 mm, in particular at least 300 mm, in particular at least 400 mm, in particular at least 500 mm and more particularly at least 600 mm.

15. Disposable nappy according to one or more of the preceding claims, **characterised in that** the hip belt (10) extends in a longitudinal direction (18) by 30 to 150 mm, in particular 40 to 130 mm, in particular 50 to 125 mm, in particular 60 to 120 mm, in particular 70 to 115 mm and more particularly 75 to 110 mm.

16. Disposable nappy according to one or more of the preceding claims, **characterised in that** the hip belt (10) is permanently joined to an outer side (12) of the main part (4).

17. Disposable nappy according to any one of the preceding claims, **characterised in that** the second closing means (88) and/or the fourth closing means (99) comprises a loop-forming non-woven material (30) as the mechanical closing element, a first upper side (40) of the non-woven material (30) which faces away from the user during use having first larger non-bonded regions (42) which are separated from one another in an insular manner, the first larger non-bonded regions (42) being limited by bonded contours (44) and surrounded by second smaller non-bonded regions (48) outside the limits and being separated from one another by the second smaller non-bonded regions (48).

18. Disposable nappy according to claim 17, **characterised in that** the second smaller non-bonded regions (48) may surround bonded partial regions (50).

19. Disposable nappy according to claim 17 or any subsequent claim, **characterised in that** each straight connecting line (56) between adjacent first larger regions (42) always extends through a bonded contour (44) or a bonded partial region (50) which is disposed between the first larger regions (42).

20. Disposable nappy according to claim 17 or any subsequent claim, **characterised in that** the first larger non-bonded insular regions (42) have a dimension, in particular a diameter of a circle (52) inscribed in the first region, of 2-15 mm, in particular 3-10 mm, and more particularly of 3-8 mm.

21. Disposable nappy according to claim 17 or any subsequent claim, **characterised in that** the first larger non-bonded insular regions (42) cover a proportion of 5-75 %, in particular 5-60 %, in particular 10-50 %, in particular 10-45 %, in particular 15-45 %, in particular 20-40 % and more particularly 30-40 % of the total surface area of the first upper side.

22. Disposable nappy according to claim 17 or any subsequent claim, **characterised in that** the first larger non-bonded insular regions (42) are circular, oval, triangular or polygonal, in particular hexagonal.

23. Disposable nappy according to claim 17 or any subsequent claim, **characterised in that** the first larger non-bonded insular regions (42) are separated from one another by at least 1 mm, in particular at least 1.5 mm, in particular at least 2 mm, in particular at least 2.5 mm, and more particularly at least 3 mm.

24. Disposable nappy according to claim 17 or any subsequent claim, **characterised in that** the total proportion of bonded regions is 10-60 %, in particular 15-40 %, in particular 15-30 %, in particular 15-25 %, in particular 19-22 % of the total surface area of the first upper surface.

25. Disposable nappy according to claim 17 or any subsequent claim, **characterised in that** loop-forming non-woven material has a stiffness of at most 0.80 N, in particular at most 0.60 N, in particular at most 0.40 N, in particular at most 0.30 N, in particular at most 0.25 N, in particular at most 0.20 N, in particular at most 0.18 N, and more particularly or at most 0.16 N, but at least 0.05 N.

## Revendications

1. Garniture jetable (2), en particulier pour les personnes incontinentes, avec une ceinture de taille (10), la ceinture de taille (10) présentant une première section de ceinture de taille (10a) et une seconde section de ceinture de taille (10b) et au moins la seconde section de ceinture de taille (10b) présentant un premier moyen de fermeture (26) sous la forme d'une languette (26) comportant des éléments de fermeture mécaniques, laquelle languette dépasse d'un bord latéral (221) de la seconde section de ceinture de taille (10b) et possède une face (261) orientée à l'opposé du corps à l'état d'utilisation et une face (262) orientée vers le corps à l'état d'utilisation, et au moins la première section de ceinture de taille (10a) présentant un deuxième moyen de fermeture (88), la face orientée vers le corps (262) du premier moyen de fermeture (26) pouvant être fixée de manière amovible sur le deuxième moyen de fermeture (88) afin de former une ouverture de taille fermée, et avec une partie principale de garniture (4) présentant une zone avant, une zone arrière et une zone d'entrejambe située entre les deux premières, laquelle partie principale de garniture présente un corps d'absorption (6) pour liquides, la partie principale de garniture (4) pouvant être fixée avec l'extrémité longitudinale de sa zone avant ou de sa zone arrière par le biais d'au moins un troisième moyen de fermeture mécanique (34) de manière amovible sur un quatrième moyen de fermeture (99) situé sur la face de la ceinture de taille (10) orientée à l'opposé du corps, **caractérisée en ce que** le troisième moyen de fermeture mécanique (34) peut être en outre fixé de manière amovible sur la face orientée à l'opposé du corps (261) du premier moyen de fermeture (26).

2. Garniture jetable selon la revendication 1, **caractérisée en ce que** la face orientée à l'opposé du corps (261) du premier moyen de fermeture (26) est constituée au moins par endroits d'une matière fibreuse (264) sur laquelle les troisièmes moyens de fermeture (34) peuvent être fixés de manière amovible.

3. Garniture jetable selon l'une des revendications précédentes, **caractérisée en ce que** le troisième moyen de fermeture (34) comporte des crochets agrippants.

4. Garniture jetable selon l'une des revendications précédentes, **caractérisée en ce que** le premier moyen de fermeture (26) est élastique au moins par endroits.

5. Garniture jetable selon l'une des revendications précédentes, **caractérisée en ce que** le premier moyen de fermeture (26) comporte des éléments de fermeture mécaniques, en particulier des crochets agrippants (263).

6. Garniture jetable selon l'une des revendications précédentes, **caractérisée en ce que** le premier moyen de fermeture (26) comporte une matière en non-tissé élastique sur laquelle le troisième moyen de fermeture (34) peut être fixé de manière amovible.

7. Garniture jetable selon l'une des revendications précédentes, **caractérisée en ce que** la force d'adhérence en tant que force de cisaillement entre la face orientée à l'opposé du corps (261) du premier moyen de fermeture (26) et le troisième moyen de fermeture (34) est plus faible que la force d'adhérence en tant que force de cisaillement entre la face orientée vers le corps (262) du premier moyen de fermeture (26) et le deuxième moyen de fermeture (88).

8. Garniture jetable selon l'une des revendications précédentes, **caractérisée en ce que** la force d'adhérence en tant que force de cisaillement entre la face orientée vers le corps (262) du premier moyen de fermeture (26) et le deuxième moyen de fermeture (88) est égale à au moins 5 N/25 mm, en particulier à au moins 10 N/25 mm, en particulier encore à au moins 15 N/25 mm, en particulier encore à au moins 20 N/25 mm, en particulier encore à au moins 25 N/25 mm, en particulier encore à au moins 30 N/25 mm, en particulier encore à au moins 35 N/25 mm, en particulier encore à au moins 40 N/25 mm, en particulier encore à au moins 45 N/25 mm et en particulier encore à au moins 50 N/25 mm, mais toutefois en particulier à au plus 80 N/25 mm, et en particulier encore à au plus 70 N/25 mm.

9. Garniture jetable selon l'une des revendications précédentes, **caractérisée en ce que** la force d'adhérence en tant que force de cisaillement entre la face orientée à l'opposé du corps (261) du premier moyen de fermeture (26) et le troisième moyen de fermeture (34) est égale à au moins 2 N/25 mm, en particulier à au moins 3,5 N/25 mm, en particulier encore à au moins 5 N/25 mm, en particulier encore à au moins 10 N/25 mm, en particulier encore à au moins 15 N/25 mm, en particulier encore à au moins 20 N/25 mm, en particulier encore à au moins 25 N/25 mm, en particulier encore à au moins 30 N/25 mm, en particulier encore à au moins 35 N/25 mm, en particulier encore à au moins 40 N/25 mm, en particulier encore à au moins 45 N/25 mm et en particulier encore à au moins 50 N/25 mm, mais toutefois en particulier à au plus 70 N/25 mm et en particulier encore à au plus 60 N/25 mm.

10. Garniture jetable selon une ou plusieurs des revendications précédentes, **caractérisée en ce que** la ceinture de taille (10) est formée par une section de matière d'un seul tenant (8) qui est assemblée sur la partie principale de garniture (4).

11. Garniture jetable selon la revendication 10 **caractérisée en ce que** la section de matière d'un seul tenant qui est assemblée sur la partie principale de garniture (4) est repliée sur elle-même des deux côtés d'un axe médian longitudinal autour de lignes de pliage (120) s'étendant dans une direction longitudinale (18) de la garniture.

12. Garniture jetable selon une ou plusieurs des revendications précédentes, **caractérisée en ce que** la ceinture de taille (10), dans sa configuration pliée, dépasse d'un bord latéral longitudinal (16) de la partie principale de garniture (4) dans le sens transversal (14) avec une zone de préhension (24) sur son extrémité libre (22) en particulier d'au moins 10 mm, en particulier d'au moins 20 mm, en particulier encore d'au moins 30 mm, en particulier encore d'au moins 40 mm, en particulier encore d'au moins 50 mm et en particulier encore d'au moins 60 mm.

13. Garniture jetable selon une ou plusieurs des revendications précédentes, **caractérisée en ce que** la ceinture de taille (10), dans sa configuration pliée, dépasse d'un bord latéral longitudinal (16) de la partie principale de garniture (4) dans le sens transversal (14) avec une zone de préhension (24) sur ses deux extrémités libres (22).

14. Garniture jetable selon une ou plusieurs des revendications précédentes, **caractérisée en ce que** l'étendue de la section de matière formant la ceinture de taille (10), à l'état dépliée, dans le sens transversal (14) au-delà du bord longitudinal (16) de la partie principale (4) est égale à au moins 200 mm, en particulier à au moins 300 mm, en particulier à au moins 400 mm, en particulier à au moins 500 mm et en particulier encore à au moins 600 mm.

15. Garniture jetable selon une ou plusieurs des revendications précédentes, **caractérisée en ce que** la ceinture de taille (10) présente une étendue dans le sens longitudinal (18) comprise entre 30 et 150 mm, en particulier entre 40 et 130 mm, en particulier entre 50 et 125 mm, en particulier entre 60 et 120 mm, en particulier entre 70 et 115 mm et en particulier encore entre 75 et 110 mm.

16. Garniture jetable selon une ou plusieurs des revendications précédentes, **caractérisée en ce que** la ceinture de taille (10) est assemblée de façon inamovible sur une face extérieure (12) de la partie principale (4).

17. Garniture jetable selon l'une des revendications précédentes, **caractérisée en ce que** le deuxième moyen de fermeture (88) et/ou le quatrième moyen de fermeture (99) comporte une matière en non-tissé formant des boucles (30) en tant que moyen de fermeture mécanique, une première face supérieure (40) de la matière en non-tissé (30) agencée de manière orientée à l'opposé du corps en utilisation comportant des premières zones assez grandes non consolidées (42) qui sont agencées espacées les unes des autres à la manière d'îlots et **en ce que** les premières zones assez grandes non consolidées (42) sont délimitées par des contours consolidés (44) et entourées à l'extérieur de la délimitation par des secondes zones plus petites non consolidées (48) et espacées les unes des autres par ces secondes zones plus petites non consolidées (48).

18. Garniture jetable selon la revendication 17, **caractérisée en ce que** les secondes zones plus petites non consolidées (48) peuvent de leur côté contenir des zones partielles (50) consolidées.

19. Garniture jetable selon la revendication 17 ou les suivantes, **caractérisée en ce que** chaque ligne de liaison droite (56) entre des premières zones assez grandes (42) voisines passe toujours par un contour consolidé (44) ou une zone partielle (50) consolidée qui est agencée entre les premières zones assez grandes (42).

20. Garniture jetable selon la revendication 17 ou les suivantes, **caractérisée en ce que** les premières zones assez grandes non consolidées à la manière d'îlots (42) présentent une dimension, en particulier un diamètre d'un cercle (52) inscrit dans la première zone, comprise entre 2 et 15 mm, en particulier entre 3 et 10 mm et en particulier encore entre 3 et 8 mm.

21. Garniture jetable selon la revendication 17 ou les suivantes, **caractérisée en ce que** les premières zones assez grandes non consolidées à la manière d'îlots (42) représentent une proportion par unité de surface comprise entre 5 et 75 %, en particulier entre 5 et 60 %, en particulier entre 10 et 50 %, en particulier entre 10 et 45 %, en particulier entre 15 et 45 %, en particulier entre 20 et 40 % et en particulier encore entre 30 et 40 %, de la surface totale de la première face supérieure.

22. Garniture jetable selon la revendication 17 ou les suivantes, **caractérisée en ce que** les premières zones assez grandes non consolidées à la manière d'îlots (42) sont réalisées circulaires ou ovales ou triangulaires ou carrées, en particulier hexagonales.

23. Garniture jetable selon la revendication 17 ou les suivantes, **caractérisée en ce que** les premières zones assez grandes non consolidées à la manière d'îlots (42) sont espacées d'au moins 1 mm, en particulier d'au moins 1,5 mm, en particulier d'au moins 2 mm, en particulier d'au moins 2,5 mm et en particulier encore d'au moins 3 mm.

24. Garniture jetable selon la revendication 17 ou les suivantes, **caractérisée en ce que** la proportion totale par unité de surface des zones consolidées est comprise entre 10 et 60 %, en particulier entre 15 et 40 %, en particulier entre 15 et 30 %, en particulier entre 15 et 25 %, en particulier entre 19 et 22 % de la surface totale de la première face supérieure.

25. Garniture jetable selon la revendication 17 ou les suivantes, **caractérisée en ce que** la matière en non-tissé formant des boucles présente une rigidité d'au plus 0,80 N, en particulier d'au plus 0,60 N, en particulier d'au plus 0,40 N, en particulier d'au plus 0,30 N, en particulier d'au plus 0,25 N, en particulier d'au plus 0,20 N, en particulier d'au plus 0,18 N, et en particulier encore d'au plus 0,16 N, mais toutefois d'au moins 0,05 N.
